# EUROPEAN PATENT SPECIFICATION

(11) **EP 0 408 668 B1**
(45) Date of publication and mention of the grant of the patent: **11.05.1994**
(21) Application number: 89906004.0
(22) Date of filing: 27.10.1988
(51) Int. Cl.: A61K 31/70, C07H 19/067

(54) **PROCESS FOR INCREASING PHOSPHATIDYLCHOLINE IN VIVO**
Verfahren zur Steigerung des Phosphatidylcholins in Vivo
PROCEDE D'AUGMENTATION DE PHOSPHATIDYLCHOLINE IN VIVO

(43) Date of publication of application: 23.01.1991
(73) Proprietor: MASSACHUSETTS INSTITUTE OF TECHNOLOGY, Cambridge, MA 02139 (US)
(72) Inventor: LOPEZ G.-COVIELLA, Ignacio, Medford, MA 02155 (US); GROWDON, John, H., Brookline, MA 02167 (US); WURTMAN, Richard, J., Boston, MA 02116 (US)
(74) Representative: Holdcroft, James Gerald, Dr.
(86) International application number: US8803827
(87) International publication number: WO9004400

(56) References cited:
- EP-A- 0 120 328
- EP-A- 0 207 436
- EP-A- 0 218 190
- DE-A- 3 732 107
- JP-A- 75 999
- JP-A- 0 108 098
- JP-A- 0 108 099
- JP-B- 7 056
- JP-B- 29 230
- US-A- 4 286 077
- US-A- 4 569 929

## Description

This invention relates to the use of cytidine or a cytidine precursor for the manufacture of a medicament for increasing the production of phosphatidylcholine (PC) by increasing a source of cytidine to a patient.

Cell membranes contain large amounts of PC, and cells must synthesize additional PC in order to divide or to change their shape as happens when there is remodeling of the synapses made by nerve cells, or neurons. In neurological diseases, when parts of a neuron have been damaged, the ability of the neuron to survive and to resume its normal communications functions requires that it synthesize relatively large amounts of additional PC, in order to make additional cellular membranes. Examples of diseases associated with damage to nerve cells include brain diseases like Alzheimer's Disease, Parkinson's Disease, strokes, the behavioral and neurological syndrome seen after brain trauma and/or anoxia, and diseases of the peripheral nervous system, e.g., neuromuscular disorders such as myasthenia gravis, the post-polio syndrome, and the muscular dystrophies.

Prior to the present invention, it has not been shown that purines or pyrimidines which are substrates in normal metabolic pathways have been rate limiting factors in producing the characteristic products of these pathways. In addition, prior to the present invention, it has not been shown that supplementary cytidine in the blood can become available to the brain for use in metabolic pathways. Cytidine is an essential substrate in the formation of PC by the following normal metabolic pathway:
Choline→Phosphocholine + Cytidyl
Triphosphate→Cytidyl
Diphosphocholine + Diacyglycerol →Phosphatidylcholine (PC)
Since PC is the product of this metabolic pathway and since it is desirable to increase its production, particularly in those areas of the human body where nerve cell damage has occurred such as in the brain, it would be highly desirable to provide a means for increasing the activity of this pathway in vivo.

Prior to the present invention, it has been shown that cytidine-diphosphocholine is useful for providing choline to form acetyl choline in vivo. This is shown, for example, in U.S. Patents 4,569,929; 4,609,647 and 4,624,852.

US-A-4,569,929 discloses the use of cytidyl diphosphocholine (CDP choline) for the treatment of disorders associated with old age, senility, Alzheimer's disease, tardive dyskenesia, Parkinson's disease, manias, ataxias, dyslexias, or neurological syndromes seen after brain trauma and/or anoxic.

DE-A-3732107 discloses the use of cytidine-diphospho-dimethyl-ethanol-amine for the treatment of cerebral disorders of vascular and traumatic origin, or disorders associated with senility, or disturbances of the intellectual capability and for the treatment of psychic stress.

EP-A-0 120 328 discloses the use of cytidine monophosphate of N-acetyl-neuraminic acid for the treatment of disturbances of the nervous stimulus in the central or peripheral nervous system.

US-A-4,386,077 discloses a pharmaceutical composition comprising CDP-choline in association with phospholipids (e.g. lecithin) for the treatment of cerebral disorders.

This invention is based upon the discovery that when cytidine is administered to a human patient, it can pass the brain-blood barrier and that the administered cytidine is capable of effecting an increase in the amount of PC which is produced by the normal metabolic pathway. Cytidine or a cytidine precursor can be administered orally such as in tablet, capsule or liquid form or parenterally by intraveneous, intramuscular or subcutaneous injection. The present invention is useful for treating patients suffering from trauma or disease associated with malformed nerve cells in the brain or periphery.

In accordance with this invention, cytidine or a cytidine precursor can be administered to a patient in order to increase brain levels of phosphatidylcholine (PC). It has been found that when cytidine is provided to neurons their production of PC can be increased, thus providing PC for forming nerve cell membranes. In addition, it has been found that the administered cytidine is converted to PC by the normal metabolic pathway which includes cytidine triphosphate and choline as substrates in the pathway. Accordingly, cytidine or a cytidine precursor can be administered alone to combine with endogenous choline in vivo. Thus, the present invention is useful for repairing damaged brain nerve cells caused by either trauma, old age or a disease state. Representative brain disease states which can be improved in accordance with this invention are Alzheimer's Disease, Parkinson's Disease, mania, ataxias including Friedreich's Ataxia, the post-stroke syndrome, the dyslexias, the behavioral and neurological syndrome seen after brain trauma and/or anoxia and those involving the peripheral nervous systems, e.g., neuromuscular disorders such as myasthenia gravis, the post-polio syndrome and the muscular dystrophies.

Representative cytidine precursors include orotic acid, or uracil-containing compounds.
The cytidine is administered so that a cytidine level of at least 4 to 200 nanomoles/ml and usually between about 6 and 50 nmoles/ml is attained in the blood stream. When the cytidine is administered in liquid form admixed with a conventional liquid carrier such as a sweetened elixer or the like from about 10 mg to 3 grams/15 ml, preferably between about 100 mg to 500 m grams/15 ml can be utilized.

The following example illustrates the present invention and is not intended to limit the same.

### EXAMPLE I

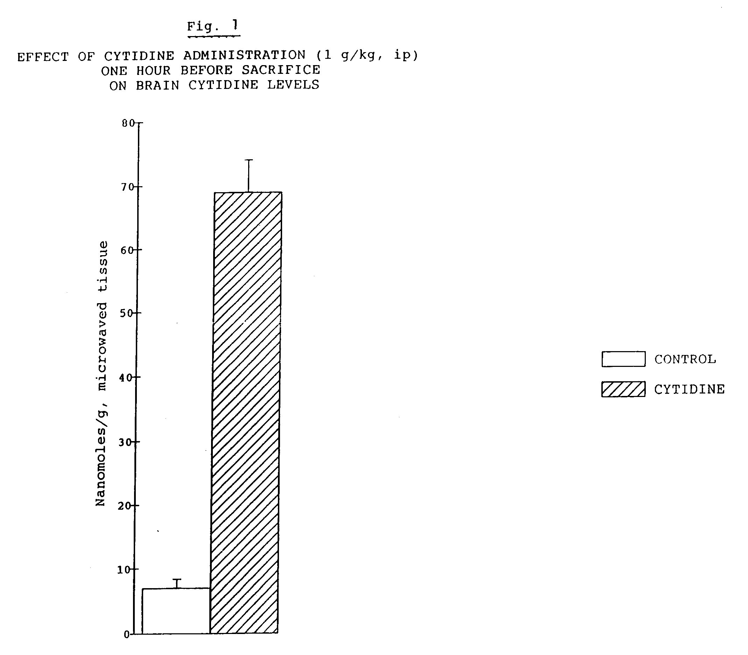

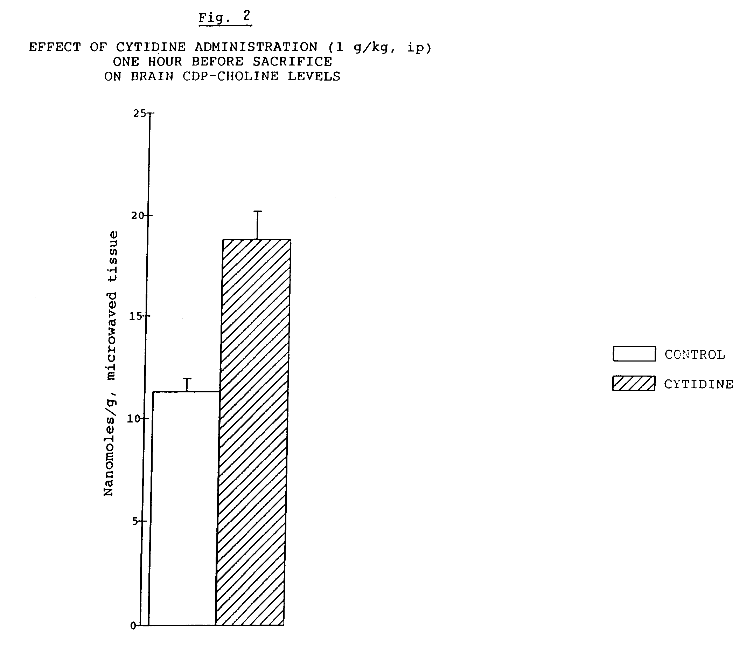

To demonstrate that administering cytidine could increase brain levels of cytidine and cytidine-(5')-diphosphate-choline (CDP-choline), the immediate precursor for phosphatidylcholine synthesis, groups of six rats received cytidine (1 g/kg, ip) or placebo and were sacrificed after 1 hour. Brain cytidine levels in treated animals rose by more than 8-fold, and brain CDP-choline levels by about 50% (Figures 1 and 2).

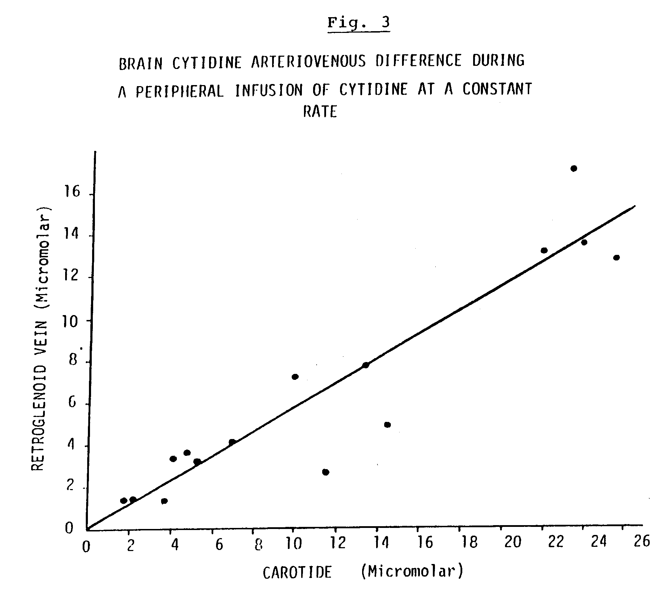

To demonstrate directly that circulating cytidine is taken up into the brain, cytidine levels were measured in carotid arterial, and retroglenoid venous blood among rats receiving a peripheral infusion of cytidine at a constant rate of .018 ml/min in the first 5 minutes, .037 ml/min for the second 5 minutes and .052 ml/min for the third 5 minutes for a total of .536 ml/15 min. The concentration of cytidine in solution was 5 mg/ml and the total dose of cytidine was 2.5 mg. Cytidine levels were found in venous blood that had drained the brain to be about 60% of the levels in arterial blood about to enter the brain, regardless of absolute levels (Figure 3).

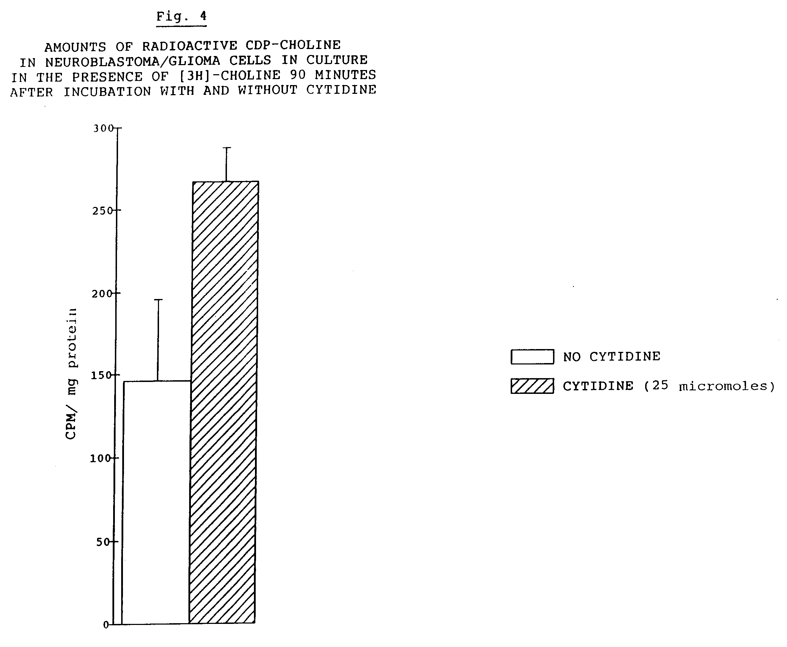

To demonstrate that an increase in cytidine levels within blood or other extracellular fluid could enhance the production of membrane phosphatidylcholine, a stable line of neuroblastoma/glioma cells (NG 108-15) were incubated with tritiated choline, and with or without supplemental cytidine (25 µM) for 90 minutes, and then the newly-formed cellular tritiated CDP-choline was extracted and measured. CDP-choline is the immediate precursor for the phosphatidylcholine in membranes of neurons and other cells and its formation controls the production of phosphatidylcholine. Cells incubated with supplemental cytidine contained almost twice as much newly-formed CDP-choline as those incubated without cytidine (Figure 4).

## Claims

1. Use of cytidine or a cytidine precursor for the manufacture of a medicament for increasing brain cytidine and blood cytidine levels (e.g. to levels of 4 to 200 nmoles/ml) in the treatment of conditions associated with damaged brain nerve cells; the medicament having cytidine or its precursor as the sole active ingredient for increasing the cytidine levels.

2. Use according to claim 1 wherein the precursor is orotic acid or an uracil-containing compound.

## Patentansprüche

1. Anwendung von Cytidin oder einer Cytidin-Vorstufe zur Herstellung eines Medikaments zur Erhöhung der Hirn-Cytidin- und Blut-Cytidingehalte (z.B. auf Gehalte von 4 bis 200 nmol/ml) bei der Behandlung von Zuständen welche im Zusammenhang mit beschädigten Hirnnervenzellen stehen, wobei das Medikament Cytidin oder seine Vorstufe als einzigen Wirkstoff zur Erhöhung des Cytidingehalts enthält.

2. Anwendung nach Anspruch 1, worin der Vorläufer Orotsäure oder eine Urazil enthaltende Verbindung ist.

## Revendications

1. Utilisation de la cytidine ou d'un précurseur de la cytidine pour la préparation d'un médicament pour augmenter les niveaux de la cytidine du cerveau et de la cytidine du sang (par exemple jusqu'à des niveaux allant de 4 à 200 nanomoles/ml) dans le traitement de conditions associées à des cellules nerveuses altérées du cerveau, le médicament comportant la cytidine ou son précurseur en tant qu'ingrédient actif unique pour l'accroissement des niveaux de la cytidine.

2. Utilisation suivant la revendication 1 caractérisée en ce que le précurseur est l'acide orotique ou un dérivé contenant un uracile.
